# EUROPÄISCHE PATENTSCHRIFT

(11) **EP 1 654 995 B1**
(45) Veröffentlichungstag und Bekanntmachung des Hinweises auf die Patenterteilung: **03.07.2013**
(21) Anmeldenummer: 05023523.3
(22) Anmeldetag: 27.10.2005
(51) Int. Cl.: A61B 17/88, A61B 17/04, A61F 2/08, B25B 15/00

(54) **Medizinisches Instrument zum Handhaben von Gegenständen, die eine Kanülierung aufweisen**
Medical instrument for handling cannulated objects
Instrument médical pour la manutention d'objets pourvus d'un alésage

(30) Priorität: 03.11.2004 DE 102004053466
(43) Veröffentlichungstag der Anmeldung: 10.05.2006
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Weller, Wolfgang

(56) Entgegenhaltungen:
- EP-A- 0 172 130
- WO-A-90/08510
- DE-A- 3 804 749
- DE-A- 10 042 424

## Beschreibung

Die Erfindung betrifft ein medizinisches Instrument zum Handhaben von Implantaten in Form von Interferenzschrauben oder Fadenankern, die eine Kanülierung aufweisen, mit einem Handgriff am proximalen Ende, mit einem als Hohlkörper ausgebildeten distalen Fortsatz, der in die Kanülierung einsteckbar ist, und mit einer Spreizvorrichtung zum radialen Spreizen des Hohlkörpers in einem Ausmaß, dass der Hohlkörper und das Implantat miteinander verpressbar sind.
Ein derartiges medizinisches Instrument ist aus der WO-A-90/08510 bekannt.

Das dort beschriebene medizinische Instrument dient zum Handhaben einer Schraube mittels eines Schraubendrehers. Die Schraube weist eine Kanülierung auf, in die ein Abschnitt des Schraubendrehers eingesteckt werden kann.

Der Abschnitt des Schraubendrehers, der in die Kanülierung eingesteckt werden kann, besteht aus unterschiedlichen konstruktiven Bauelementen mit unterschiedlichen konstruktiven Zwecken. Von proximal nach distal gesehen weist der in die Kanülierung einzuführende Abschnitt einen ersten Bereich auf, der nicht spreizbar ist und der der Kontur der Kanülierung angepasst ist. Dieser Abschnitt ist als Sechskant ausgebildet, der in einen entsprechenden Innensechskant der Kanülierung im Passsitz eingeschoben werden kann. Dieser Abschnitt ist der Kraft übertragende Abschnitt, d.h. die Kräfte werden über diesen Abschnitt vom Handhabungsgerät auf die Schraube beim Drehen übertragen.

Dieser Sechskantabschnitt setzt sich in einem mit Längsschlitzen versehenen, radial spreizbaren Körper fort, an dessen distalem Ende ein durchmessererweiterter, distaler, kurzer Fortsatz vorgesehen ist, dessen Außendurchmesser etwas größer ist als der lichte Innendurchmesser der Kanülierung. Dadurch wird der spreizbare Abschnitt beim Einführen in die Kanülierung etwas radial nach innen zusammengepresst. Durch die elastische Ausbildung hat der zusammengepresste Fortsatz die Tendenz, sich wieder aufzuweiten, übt somit auf die Innenwand der Kanülierung eine gewisse Anpresskraft aus, die dafür sorgt, dass bspw. der Schraubendreher nicht von der Schraube oder umgekehrt abfällt, und erleichtert somit die Handhabung.

In einer Ausgestaltung ist vorgesehen, durch die gesamte Vorrichtung, also sowohl durch den Schraubendreher als auch die Schraube hindurch, einen Zieldraht, einen so genannten "K"-Draht hindurch zu schieben.

Die Innenseite der Kanülierung im Bereich des distalen, durchmessererweiternden Fortsatzes ist nun so ausgebildet, dass sie sich etwas konisch verjüngt, somit durch einen eingeschobenen Zieldraht radial gespreizt wird. Dadurch wird bei eingeschobenem Zieldraht dieser distale Fortsatz noch verstärkt an die Innenwandung der Kanülierung gepresst. Auch diese Maßnahme ist eine zusätzliche Sicherungsmaßnahme für die Handhabung. Die Kraft zum Ein- oder Ausdrehen wird über den Abschnitt mit dem Sechskant übertragen.

Gegenstände, die eine Kanülierung aufweisen, sind bspw. Implantate, die im menschlichen Körper, insbesondere in einem Knochen, eingesetzt werden. Gebräuchliche Implantate, die als sogenannte Interferenzschrauben ausgebildet sind, dienen dazu, einen Sehnenersatz, insbesondere einen Kreuzbandersatz, im Kniebereich zu fixieren. Dazu wird in den Oberschenkel- bzw.

Unterschenkenknochen eine entsprechende Bohrung eingebracht in die der Sehnenersatz eingeschoben wird. Zum Fixieren der Sehne wird in den Freiraum zwischen Sehne und Wand der Bohrung eine sogenannte Interferenzschraube eingedreht, die die Sehne seitlich an die Wand der Bohrung presst und dadurch fixiert.

Andere Gegenstände sind bspw. Fadenanker, die in Knochenbohrungen eingesetzt werden, um Halte- oder Fixationsfäden zu fixieren.

All diesen Gegenständen ist gemein, dass sie eine Kanülierung aufweisen. Diese Kanülierung kann als durch die gesamte axiale Länge durchgehender Kanal ausgebildet sein, bspw. um Fixationsfäden durch den Körper hindurchfädeln zu können, oder die Kanülierung kann lediglich in Form einer Einsenkung bestehen, um ein Werkzeug, bspw. einen Schraubendreher anzusetzen, um den Gegenstand, wenn er als Interferenzschraube ausgebildet ist, drehen zu können.

Die Kanülierung kann jegliche Querschnittsform aufweisen, also einen runden Querschnitt oder bspw. einen sechskantigen oder sternförmigen Querschnitt.

Es ist bekannt geworden, derartige Interferenzschrauben entweder aus metallischen Materialien, insbesondere aus Titan, herzustellen oder auch aus biodegradierbaren Materialien, siehe dazu Sonderveröffentlichung OP-Journal, Nr. 3/Jahrgang 14/Dezember 1998, A. Weiler et al. "Biodegradierbare Interferenzschrauben in der Kreuzbandchirurgie".

Eine Operationstechnik, insbesondere der zuvor erwähnte Vorgang des Fixierens eines Sehnentransplantates ist bspw. aus der EP 1 093 773 A1 der Anmelderin beschrieben.

Im praktischen Einsatz wurde festgestellt, dass beim Fixieren derartiger Gegenstände relativ hohe Kräfte einwirken, die dazu führen können, dass die entsprechenden Schlitze oder Innensechskants, an denen ein Drehwerkzeug angesetzt wird, ausbrechen oder deformiert werden.

Ein weiteres Problem ergibt sich insbesondere in der Kreuzbandchirurgie bei einem sogenannten Revisionseingriff, d.h. wenn das Implantat wieder entfernt werden muss, weil bspw. die Sehne erneut gerissen ist.

Dabei stellt die Bergung der vom Voroperateur verwendeten Implantate eine besondere Herausforderung dar. Dies liegt darin begründet, dass die Implantate mit dem umgebenden Gewebe und Knochen verwachsen sind und die Schraubenköpfe schon bei Einbringen deformiert worden sind.

Es ist daher Aufgabe der vorliegenden Erfindung, ein medizinisches Instrument zum Handhaben von derartigen Gegenständen bereitzustellen, das eine möglichst weitgehend zerstörungsfreie Handhabung derartiger Gegenstände erlaubt, insbesondere es ermöglicht, solche Gegenstände bei einem Revisionseingriff zu bergen.

Erfindungsgemäß wird die Aufgabe dadurch gelöst, dass der Hohlkörper durch die Spreizvorrichtung derart radial aufweitbar ist, dass die ganze äußere Oberfläche des Hohlkörpers zur Bildung einer Flächenpressung herangezogen wird.

Der distale Fortsatz kann nunmehr in die Kanülierung des zu handhabenden Gegenstandes eingesteckt werden. Dazu weist der Fortsatz eine entsprechende Länge und einen entsprechenden Durchmesser auf.

Durch die Spreizvorrichtung kann der distale Fortsatz, nachdem er in die Kanülierung eingeschoben wurde, radial aufgeweitet werden, wodurch er flächig und unter Druck an der Innenwand der Kanülierung zum Liegen kommt, somit unter einer Flächenpressung im zu handhabenden Gegenstand sitzt.

Dadurch wird über eine relativ große Fläche ein fester kraftschlüssiger Sitz zwischen Gegenstand und dem medizinischen Instrument geschaffen, der eine Handhabung ermöglicht.

Die Handhabung kann sowohl das Eindrehen oder das Einsetzen des Gegenstandes als auch das Bergen des Gegenstandes betreffen.

Ein besonderer Vorteil der Spreizvorrichtung besteht darin, dass nicht wie bei Werkzeugen üblich ein Formschluss geschaffen wird, sondern dass durch die Spreizung ein unter Flächenpressung fester Sitz zum Handhaben erzeugt wird.

Dies ermöglicht bspw. beim Bergen von Interferenzschrauben, die schon relativ fest mit dem Knochenmaterial verwachsen sind, eine feste Verbindung zwischen dem Instrument und dem Gegenstand herzustellen, um auch entsprechend hohe Kräfte einwirken zu lassen, um eine solche verwachsene Schraube ausdrehen zu können.

Durch den relativ großflächigen Sitz in der Kanülierung spielt es keine Rolle, ob bspw. eine Einsenkung in Form eines Sechskantes beim Eindrehen der Schraube schon deformiert wurde, durch die Spreizung wird ein ausreichend fester Sitz gewährleistet.

Je nach dem, welches Material der zu bergende Gegenstand aufweist, sei es Metall oder Kunststoff, kann auch das Material ausgewählt werden, aus der die Vorrichtung hergestellt ist.

Bestehen bspw. in der Kanülierung relativ starke geometrische Verformungen, kann das Material des distalen Fortsatzes relativ weich ausgebildet sein, so dass es durch die Spreizvorrichtung sich mit den Verformungen verkrallt, so dass ein besonders großer Flächeneingriff möglich ist. Das Material kann auch aus Metall bestehen, um bei bspw. aus Kunststoff bestehenden Interferenzschrauben eventuelle Auswüchse oder Einwachsungen im Bereich der Kanülierung zu Glätten, um so zu einem großflächigen Presssitz zu kommen.

Letztendlich kann ein derartiges medizinisches Instrument bei allen Arten von kanülierten Implantaten Einsatz finden, wobei bevorzugt ein Einsatz eine Bergung eines Implantats im Rahmen eines Revisionseingriffes darstellt. Die Vorrichtung kann aber auch zur primären Implantation verwendet werden. Es ist nicht mehr notwendig, das Implantat zum Bergen herauszubohren, wobei bspw. bei metallischen Implantaten ein Metallabrieb entsteht, der sorgfältig entfernt werden muss. Es sind nunmehr auch sogenannte Riesendefekte durch Überbohren der Implantate ausgeschlossen.

In einer weiteren Ausgestaltung der Erfindung weist die Spreizvorrichtung einen Spreizdorn auf.

Diese Maßnahme hat den Vorteil, dass der distale Fortsatz zunächst in ungespreiztem Zustand in die Kanülierung eingebracht werden kann, und anschließend durch den Spreizdorn aufgeweitet werden kann.

Dabei ist der Spreizdorn als Konus ausgebildet.

Diese Maßnahme hat den Vorteil, dass diese Konusgeometrie gleichmäßig über die Konusfläche einen radial aufweitenden Anpressdruck erzeugen kann.

Ferner ist die Innenseite des Hohlkörpers entsprechend konisch ausgebildet.

Diese Maßnahme hat den Vorteil, dass, sobald die konischen Flächen gegeneinander anlaufen, der distale Fortsatz etwas aufgespreizt wird und dies zu dem "Pressfit" in der Kanülierung über einen großen Flächenbereich führt.

In einer weiteren Ausgestaltung der Erfindung weist der Spreizdorn eine das distale Ende des Fortsatzes überragende Verlängerung auf.

Diese Maßnahme hat den Vorteil, dass diese Verlängerung als eine Art Führungsdraht oder Einführhilfe zum Einbringen des Instrumentes in die Kanülierung des zu handhabenden Gegenstandes darstellt.

In einer weiteren Ausgestaltung der Erfindung ist proximal ein Handgriff mit einer Handhabe zum Betätigen der Spreizvorrichtung vorgesehen.

Diese Maßnahme erleichtert die Handhabung des medizinischen Instrumentes. Es kann bspw. wie ein Schraubendreher von einer Hand ergriffen werden, und zunächst in die Kanülierung des zu handhabenden Gegenstandes angesetzt und eingeschoben werden, und anschließend wird über die Handhabe am Griff die Spreizvorrichtung bedient.

In einer weiteren Ausgestaltung der Erfindung ist dazu vorteilhaft, dass die Handhabe einen Hebel aufweist, über den der Spreizdorn axial im Fortsatz verschiebbar ist.

Auch diese Maßnahme erleichtert die Handhabung. Nach dem Ansetzen und Einbringen in die Kanülierung wird der Hebel verschwenkt, dabei wird der Spreizdorn nach distal verschoben und sorgt für die Aufweitung. Aus der Schwenkstellung des Hebels kann die Handhabungsperson auch jeweils erkennen, in welcher Position sich die Spreizvorrichtung befindet.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine Seitenansicht eines erfindungsgemäßen medizinischen Instrumentes im Bereich des distalen Fortsatzes,
- Fig. 2: einen Längsschnitt der Darstellung von Fig. 1,
- Fig. 3: einen Querschnitt längs der Linie III-III in Fig. 2,
- Fig. 4: eine der Schnittdarstellung von Fig. 2 vergleichbare, etwas vergrößerte Darstellung, bei der gerade ein Dorn der Spreizvorrichtung zum Spreizen eingefahren wird,
- Fig. 5: eine weitere Ausgestaltung eines derartigen Spreizdorns mit einer distalen Verlängerung,
- Fig. 6: stark schematisiert das medizinische Instrument kurz vor dem Einbringen in eine Kanülierung einer Fixationsschraube, die in einem Knochen eingebracht ist, und
- Fig. 7: eine der Fig. 6 vergleichbare Stellung nach Einbringen des distalen Fortsatzes in die Kanülierung der Schraube und Betätigen der Spreizvorrichtung zur Schaffung der Presssitzverbindung zwischen Instrument und zu bergendem Gegenstand.

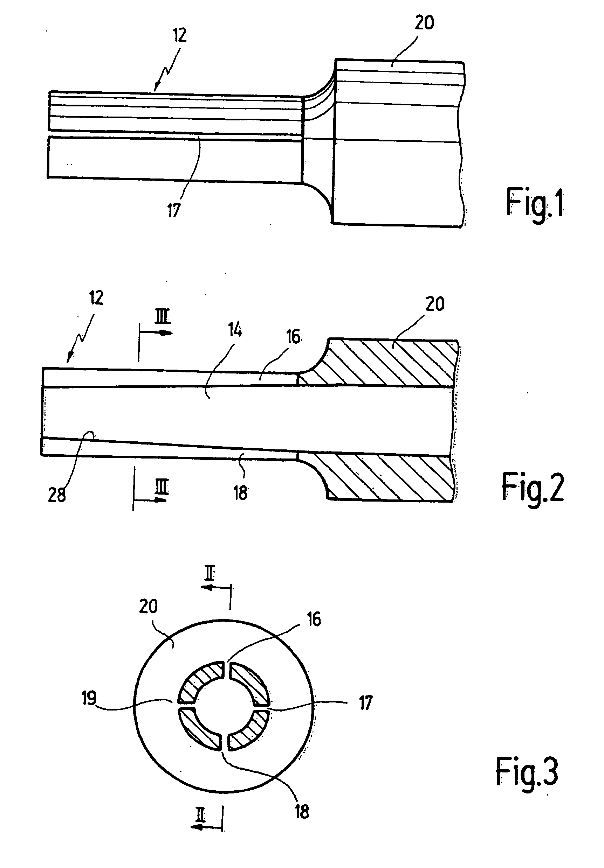

Ein in den Figuren 1 bis 7 dargestelltes medizinisches Instrument ist in seiner Gesamtheit mit der Bezugsziffer 10 bezeichnet.

Wie insbesondere aus den Figuren 1 bis 3 zu entnehmen, weist das medizinische Instrument 10 eine distalen Fortsatz 12 auf, der als Hohlkörper 14 ausgebildet ist.

Der Hohlkörper 14 ist umfänglich gleichmäßig verteilt mit vier axial bis zur distalen Spitze durchgehenden Schlitzen 16, 17, 18, 19 versehen.

Der distale Fortsatz 12 ist mit einem hohlzylindrischen Körper 20 mit größerem Durchmesser verbunden.

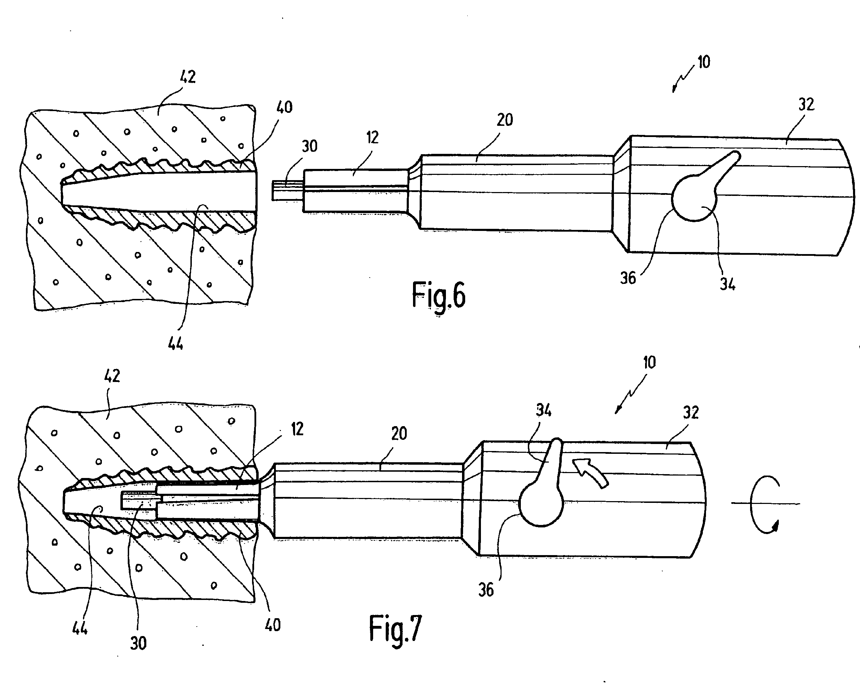

Wie insbesondere aus Fig. 6 und 7 zu erkennen, geht der Körper 20 in einen noch weiter durchmessergrößeren, etwa zylindrisch ausgebildeten Handgriff 32 am proximalen Ende über.

Im Inneren des medizinischen Instrumentes 10, das durchgehend als Hohlkörper ausgebildet ist, ist ein Spreizdorn 22 aufgenommen. Der Spreizdorn 22 weist einen zylindrisch stangenförmigen Körper 24 auf, dessen distaler Endbereich als Konus 26 ausgebildet ist.

Die Innenseite 28 des Hohlkörpers 14 ist komplementär konisch zu dem Konus 26 ausgebildet.

Der stangenförmige Körper 24 erstreckt sich bis in den Handgriff 32 hinein, und steht dort mit einem Hebel 34 in Wirkverbindung, über den der Spreizdorn 22 axial hin und her verschiebbar ist.

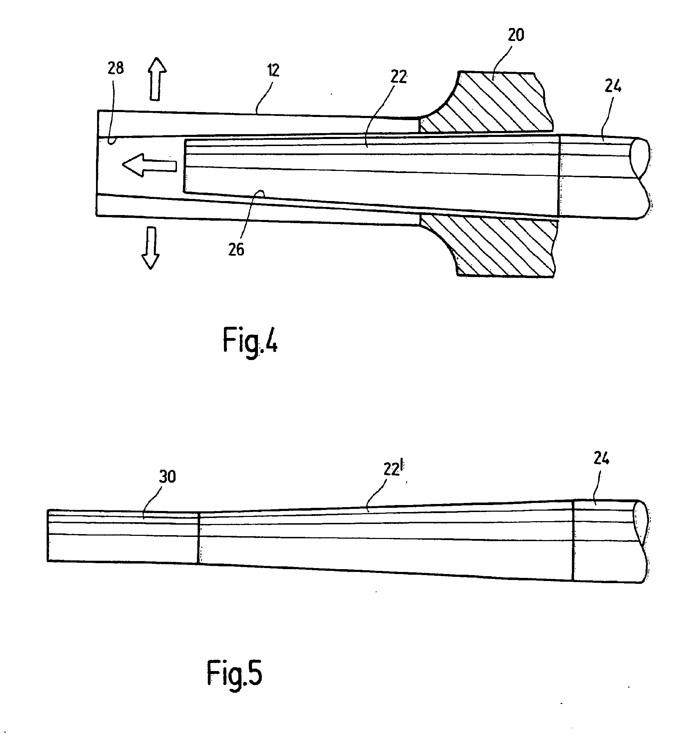

In Fig. 5 ist ein weiteres Ausführungsbeispiel eines Spreizdorns 22' dargestellt, der sich vom Spreizdorn 22 lediglich dadurch unterscheidet, dass sein distales Ende durch einem Führungsstab 30 bzw. Führungsdraht verlängert ist. Die Länge dieses Führungsabschnittes ist dabei so, dass dieser das distale Ende des distalen Fortsatzes 12 überragt, wie das aus Fig. 6 ersichtlich ist.

Die Bauteile Hebel, Spreizdorn stellen eine Spreizvorrichtung 36 dar, die dazu dient, den Hohlkörper 14 radial zu spreizen.

Wie insbesondere aus Fig. 4 zu erkennen, treten der Konus 26 und die entsprechend konisch ausgebildete Innenseite 28 des Hohlkörpers 14 miteinander in Eingriff, wonach ein weiteres Vorschieben des Spreizdornes 22 nach distal für eine radiale Aufweitung bzw. Aufspreizung des Hohlkörpers 14 sorgt, wie das in Fig. 4 durch die Pfeile dargestellt ist. Dieses Spreizen oder Aufweiten wird insbesondere dadurch erleichtert, dass der Hohlkörper 14 durch die Schlitze 16 bis 19 in vier Segmente aufgeteilt ist, wie das insbesondere aus der Schnittdarstellung von Fig. 3 ersichtlich ist.

Im Zusammenhang mit Fig. 6 und 7 soll nunmehr ein praktischer Anwendungsfall dargestellt werden, wobei hier das medizinische Instrument 10 dazu dient, einen Gegenstand 40 in Form einer Interferenzschraube zu bergen, die in einem Knochen 42 eingedreht wurde. Der Gegenstand 40 weist mittig eine zylindrische Kanülierung 44 auf.

In diesem Fall ist die Geometrie des distalen Fortsatzes 12 ebenfalls zylindrisch, wobei der Außendurchmesser des Hohlkörpers 14 im ungespreizten Zustand in etwa dem lichten Innendurchmesser der Kanülierung 44 entspricht.

Bei entsprechend anderen Dimensionierungen oder entsprechend anderen Geometrien sind dann die Geometrie von distalem Forsatz 12 und Kanülierung 44 entsprechend aneinander angepasst.

In der in Fig. 6 dargestellten Position befindet sich die Spreizvorrichtung 36 in ihrer nicht spreizenden Stellung, d.h. der Spreizdorn 22 liegt gerade an der inneren konischen Fläche des Hohlkörpers 14 an.

Der Führungsstab oder Führungsdraht 30 überragt den Hohlkörper 14 etwas und dient zum Erleichtern des Ansetzens und Einfädelns oder Einbringens des distalen Fortsatzes 12 in die Kanülierung des zu bergenden Gegenstandes 40.

Nachdem der Hohlkörper 14 vollständig in die Kanülierung 44 eingeschoben ist, wird der Hebel 34 verschwenkt, wobei der Spreizdorn 22 nach distal verschoben wird und die Segmente des Hohlkörpers 14 radial spreizt und aufweitet, so dass ein fester Flächenpresssitz zwischen den gespreizten Segmenten des Hohlkörpers 14 und der Innenwand der Kanülierung 44 des zu bergenden Gegenstandes 40 bewerkstelligt wird.

Nunmehr kann dieser Zusammenbau gedreht werden, bspw. entgegen dem Uhrzeigersinn, um die Fixationsschraube aus dem Knochen herauszudrehen, um sie dadurch zu bergen.

Es ist selbstverständlich einleuchtend, dass ein solcher Zusammenbau auch zum Eindrehen einer solchen Schraube eingesetzt werden kann. In diesem Fall wird die Schraube bzw. deren Kanülierung auf den Hohlkörper 14 aufgeschoben, die Spreizvorrichtung betätigt und dann dieser Zusammenbau in eine entsprechende Bohrung angesetzt, um die Schraube einzudrehen.

Die Spreizvorrichtung wird dann wieder entsprechend betätigt, so dass das medizinische Instrument 10 anschließend abgenommen werden kann.

## Patentansprüche

1. Medizinisches Instrument zum Handhaben von Implantaten in Form von Interferenzschrauben (40) oder Fadenankem, die eine Kanülierung (44) aufweisen, mit einem Handgriff (32) am proximalen Ende, mit einem als Hohlkörper (14) ausgebildeten distalen Fortsatz (12), der in die Kanülierung (44) einsteckbar ist, und mit einer Spreizvorrichtung (36) zum radialen Spreizen des Hohlkörpers (14) in einem Ausmaß, dass der Hohlkörper (14) und das Implantat miteinander verpressbar sind, **dadurch gekennzeichnet, dass** der Hohlkörper (14) durch die Spreizvorrichtung (36) derart radial aufweitbar ist, dass die ganze äußere Oberfläche des Hohlkörpers (14) zur Bildung einer Flächenpressung herangezogen wird.

2. Medizinisches Instrument nach Anspruch 1, **dadurch gekennzeichnet, dass** die Spreizvorrichtung (36) einen Spreizdorn (22, 22') mit einem Konus (26) aufweist, und dass die Innenseite (28) des Hohlkörpers (14) entsprechend konisch ausgebildet ist.

3. Medizinisches Instrument nach Anspruch 2, **dadurch gekennzeichnet, dass** der Spreizdorn (22') eine das distale Ende des Hohlkörpers (14) überragende Verlängerung (30) aufweist.

4. Medizinisches Instrument nach einem der Ansprüche 1 bis 3, **dadurch gekennzeichnet, dass** der Handgriff (32) eine Handhabe zum Betätigen der Spreizvorrichtung (36) aufweist.

5. Medizinisches Instrument nach Anspruch 4, **dadurch gekennzeichnet, dass** die Handhabe einen Hebel (34) aufweist, über den ein Spreizdorn (22, 22') axial im Hohlkörper (14) verschiebbar ist.

## Claims

1. Medical instrument for manipulating of implants in form of interference screws (40) or thread anchors which have a hollow channel (44), comprising a handle (32) at the proximal end, a distal tip (12) designed as a hollow body (14) which can be inserted into the hollow channel (44), and with an expanding device (36) for radially expanding the hollow body (14) to such an extent that the hollow body (14) and the implant can be pressed tightly together, **characterized in that** the hollow body (14) can be expanded radially by the expanding device (36) **in that** the entire outer sur-face of the hollow body (14) is used for making a surface pressure.

2. Medical instrument of claim 1, **characterized in that** the expanding device (36) has an expanding pin (22, 22') provided with a cone (26), and **in that** the inner face (28) of the hollow body (14) has a corresponding conical shape.

3. Medical instrument of claim 2, **characterized in that** the expanding pin (22) has a prolongation (30) extending beyond the distal end of the hollow body (14).

4. Medical instrument of any one of claims 1 through 3, **characterized in that** the handle (32) has a manipulator for manipulating the expanding device (36).

5. Medical instrument of claim 4, **characterized in that** the manipulator has a lever (34) via which the expanding pin (22, 22') can be displaced axially within the hollow body (14).

## Revendications

1. Instrument médical pour manipuler des implants sous la forme de vis d'interférence (40) ou d'ancrages de fil qui présentent un alésage (44), avec une poignée (32) à l'extrémité proximale, avec un prolongement distal (12), qui est réalisé sous forme de corps creux (14) et qui peut être enfilé dans l'alésage (44), et avec un dispositif d'expansion (36) pour l'expansion radiale du corps creux (14) dans une mesure telle que le corps creux (14) et l'implant peuvent être mutuellement assemblés par pressage, **caractérisé en ce que** le corps creux (14) peut être élargi radialement par le dispositif d'expansion (36) de telle sorte que la totalité de la surface extérieure du corps creux (14) est utilisée pour former une pression superficielle.

2. Instrument médical selon la revendication 1, **caractérisé en ce que** le dispositif d'expansion (36) présente un mandrin d'expansion (22, 22') doté d'un cône (26), et **en ce que** le côté intérieur (28) du corps creux (14) est réalisé avec une conicité correspondante.

3. Instrument médical selon la revendication 2, **caractérisé en ce que** le mandrin d'expansion (22') présente un prolongement (30) dépassant de l'extrémité distale du corps creux (14).

4. Instrument médical selon l'une des revendications 1 à 3, **caractérisé en ce que** la poignée (32) présente une manette pour actionner le dispositif d'expansion (36).

5. Instrument médical selon la revendication 4, **caractérisé en ce que** la manette présente un levier (34) au moyen duquel un mandrin d'expansion (22, 22') est axialement mobile dans le corps creux (14).
